# EUROPEAN PATENT APPLICATION

(11) **EP 1 103 610 A1**
(43) Date of publication of application: **30.05.2001**
(21) Application number: 99203983.4
(22) Date of filing: 26.11.1999
(51) Int. Cl.: C12N 15/34, C12N 5/10, C07K 14/11, C07K 14/075, C12N 15/85, C12N 7/02, A61K 39/145

(54) **Production of vaccines from immortalised mammalian cell lines**

(71) Applicant: Introgene B.V., 2333 AL Leiden (NL)
(72) Inventor: Pau, Maria Grazia, 2311 SK Leiden (NL); UytdeHaag, Alphonsus Gerardus Cornelis Maria, 3731 EP De Bilt (NL)
(74) Representative: Ottevangers, Sietse Ulbe

(57) **Abstract**

Novel means and methods are provided for the production of mammalian viruses, comprising infecting a culture of immortalized human cells with the virus, incubating the culture infected with virus to propagate the virus under conditions that permit growth of the virus, and to form a virus-containing medium, and removing the virus-containing medium.

The viruses can be harvested and be used for the production of vaccines.

Advantages - human cells of the present invention can be cultured under defined serum free conditions, and the cells show improved capability for propagating virus

In particular, methods are provided for producing in cultured human cells influenza virus and vaccines derived thereof. This method eliminates the necessity to use whole chicken embryos for the production of influenza vaccines.

The method provides also for the continuous or batchwise removal of culture media. As such, the present invention allows the large scale continuous production of viruses to a high titer.

## Description

### FIELD OF THE INVENTION

The invention relates to the development and manufacturing of vaccines. In particular the invention relates to the field of production of viral proteins and/or viruses, more in particular to the use of a mammalian cell, preferably a human cell for the production of viruses growing in eukaryotic, preferably mammalian and in particular human cells. The invention is particularly useful for the production of vaccines to aid in protection against viral pathogens for vertebrates, in particular mammalians and especially humans. Means and methods are disclosed herein for producing a virus and/or viral protein in a (human) cell, preferably using a defined synthetic medium, and for purifying the virus and/or components thereof from the cell and/or culture medium. Pharmaceutical compositions containing virus or its components and methods for manufacturing and recovering and/or purifying them are provided.

### BACKGROUND

Vaccination is the most important route of dealing with viral infections. Although a number of antiviral agents is available, typically these agents have limited efficacy. Administering antibodies against a virus may be a good way of dealing with viral infections once an individual is infected (passive immunisation) and typically human or humanised antibodies do seem promising for dealing with a number of viral infections, but the most efficacious and safe way of dealing with virus infection is and probably will be prophylaxis through active immunisations. Active immunisation is generally referred to as vaccination and vaccines comprise at least one antigenic determinant of a virus, preferably a number of different antigenic determinants of at least one virus, e.g by incorporating in the vaccine at least one viral polypeptide or protein derived from a virus (subunit vaccins). Typically the formats mentioned sofar include adjuvants in order to enhance an immune response. This also is possible for vaccines based on whole virus, e.g. in an inactivated format. A further possibility is the use of live, but attenuated forms of the pathogenic virus. A further possibility is the use of wild-type virus, e.g in cases where adult individuals are not in danger from infection, but infants are and may be protected through maternal antibodies and the like. Production of vaccines is not always an easy procedure. In some cases the production of viral material is on eggs, which leads to difficult to purify material and extensive safety measures against contamination, etc. Also production on bacteria and or yeasts, which sometimes but not always is an alternative for eggs requires many purification and safety steps. Production on mammalian cells would be an alternative, but mammalian cells used so far all require for instance the presence of serum and/or adherence to a solid support for growth. In the first case again purification and safety and e.g. the requirement of protease to support the replication of some viruses become an issue. In the second case high yields and ease of production become a further issue. The present invention overcomes at least a number of the problems encountered with the production systems for production of viruses and/or viral proteins for vaccine purposes of the systems of the prior art.

### DESCRIPTION OF THE INVENTION

Thus the invention provides a method for producing a virus and/or viral proteins other than adenovirus or adenoviral proteins for use as a vaccine comprising providing a cell with at least a sequence encoding at least one gene product of the E1 gene or a functional derivative thereof of an adenovirus, providing said cell with a nucleic acid encoding said virus or said viral proteins, culturing said cell in a suitable medium and allowing for propagation of said virus or expression of said viral proteins and harvesting said virus and/or viral proteins from said medium and/or said cell.

Until the present invention there are few, if any (human) cells that have been found suitable to produce viruses and/or viral proteins for use as vaccines in any reproducible and upscaleable manner and/or sufficiently high yields and/or easily purifiable. We have now found that cells which comprise adenoviral E1 sequences, preferably in their genome are capable of sustaining the propagation of viruses in significant amounts.
The preferred cell according to the invention is derived from a human primary cell, preferably a cell which is immortalised by a gene product of said E1 gene. In order to be able to grow a primary cell of course needs to be immortalized. A good example of such a cell is one derived from a human embryonic retinoblast.

In cells according to the invention it is important that the E1 gene sequences are not lost during the cell cycle. It is therefore preferred that said sequence encoding at least one gene product of the E1 gene is present in the genome of said (human) cell. For reasons of safety care is best taken to avoid unnecessary adenoviral sequences in the cells according to the invention. It is thus another embodiment of the invention to provide cells that do not produce adenoviral structural proteins. However, in order to achieve large scale (continuous) virus production through cell culture it is preferred to have cells capable of growing without needing anchorage. The cells of the present invention have that capability. To have a clean and safe production system from which it is easy to recover and, if desirable, to purify the virus, it is preferred to have a method according to the invention, whereby said human cell comprises no other adenoviral sequences. The most preferred cell for the methods and uses of the invention is PER.C6 as deposited under ECACC no. 96022940, or a derivative thereof.

Thus the invention provides a method using a cell according to the invention, wherein said cell further comprises a sequence encoding E2A or a functional derivative or analogue or fragment thereof, preferably a cell wherein said sequence encoding E2A or a functional derivative or analogue or fragment thereof is present in the genome of said human cell and most preferably a cell wherein said E2A encoding sequence encodes a temperature sensitive mutant E2A.

Furthermore, as stated the invention also provides a method according to the invention wherein said (human) cell is capable of growing in suspension.

The invention also provides a method wherein said human cell can be cultured in the absence of serum. The cells according to the invention, in particular PER.C6 has the additional advantage that it can be cultured in the absence of serum or serum components. Thus isolation is easy, safety is enhanced and reliability of the system is good (synthetic media are the best in reproduceability). The human cells of the invention and in particular those based on primary cells and particularly the ones based on HER cells, are capable of normal post and peritranslational modifications and assembly. This means that they are very suitable for preparing viral proteins and viruses for use in vaccines.

Thus the invention provides a method according to the invention, wherein said virus and/or said viral proteins comprise a protein that undergoes post-translational and/or peritranslational modification, especially wherein said modifications comprise glycosylation. A good example of a viral vaccine that has been cumbersome to produce in any reliable manner is influenza vaccine. The invention provides a method according the invention wherein said viral proteins comprise at least one of an Influenza virus neuramidase and/or a haemagglutinin. Other viral proteins (subunits) and viruses (wild-type to be inactivated) or attenuated viruses that can be produced in the methods according to the invention include enterovirus, such as rhinovirus, aphtovirus, or poliomyelitisvirus, herpesvirus, such as herpes symplex virus, pseudorabies virus or bovine herpes virus, orthomyxovirus, such as influenza virus, a paramyxovirus, such as newcastle disease virus, respiratory syncitio virus, mumps virus or a measles virus, retrovirus, such as human immunedeficiency virus or a parvovirus or a papovavirus, rotavirus or a coronavirus, such as transmissable gastroenteritisvirus or a flavivirus, such as tick-borne encephalitis virus or yellow fever virus, a togavirus, such as rubella virus or eastern-, western-, or venezuelean equine encephalomyelitis virus, a hepatitis causing virus, such as hepatitis A or hepatitis B virus, a pestivirus, such as hog cholera virus or a rhabdovirus, such as rabies virus.

The invention also provides the use of a human cell having a sequence encoding at least one E1 protein of an adenovirus or a functional derivative, homologue or fragment thereof in its genome which cell does not produce structural adenoviral proteins for the production of a virus, or at least one viral protein for use in a vaccine. Of course for such a use the cells preferred in the methods according to the invention are also preferred. The invention also provides the products resulting from the methods and uses according to the invention, especially viral proteins and viruses obtainable according to those uses and/or methods, especially when brought in a pharmaceutical composition comprising suitable excipients and in some formats (inactivated viruses, subunits) adjuvants. Dosage and ways of administration can be sorted out through normal clinical testing in as far as they are not yet available through the already registered vaccines.

Thus the invention also provides a virus or a viral protein for use in a vaccine obtainable by a method or by a use according to the invention, said virus or said viral being free of any non-human mammalian protenaceous material and a pharmaceutical formulation comprising such a virus and/or viral protein.

The invention further provides a human cell having a sequence encoding at least one E1 protein of an adenovirus or a functional derivative, homologue or fragment thereof in its genome, which cell does not produce structural adenoviral proteins and having a nucleic acid encoding a virus or at least one non-adenoviral viral protein. This cell can be used in a method according to the invention.

In a preferred embodiment the invention provides influenza virus obtainable by a method according to the invention or by a use according to the invention. In another embodiment the invention provides influenza vaccines obtainable by a method according to the invention or by a use according to the invention.

### Detailed description.

The present invention discloses a novel human immortalized cell line for the purpose of propagating and harvesting virus, for production of said virus. PER.C6 cells (WO 97/00326) were generated by transfection of primary human embryonic retina cells, using a plasmid that contained the Ad serotype 5 (Ad5) E1A- and E1B-coding sequences (Ad5 nucleotides 459-3510) under the control of the human phosphoglycerate kinase (PGK) promoter.

The following features make PER.C6 or a derivative particularly useful as a host for virus production: it is a fully characterized human cell line, it was developed in compliance with GLP, it can be grown as suspension cultures in defined serum-free medium, devoid of any human or animal serum proteins; its growth is compatible with roller bottles, shaker flasks, spinner flasks and bioreactors, with doubling times of about 35 hrs;

### Influenza epidemiology.

Influenza viruses, members of the family of Orthomyxoviridae, are the causative agents of annual epidemics of acute respiratory disease. In the US alone 50 million Americans get flu each year. Estimated deaths worldwide (1972-1992) are 60.000 (CDC statistics). There have been 3 major cases of pandemics outbreaks of influenza, namely in 1918 (Spanish flu, est. 40 million deaths), in 1957 (Asian flu, est. 1 million deaths), and in 1968 (Hong-Kong flu, est. 700.00 deaths). Infections with influenza viruses are associated with a broad spectrum of illnesses and complications that result in substantial worldwide morbidity and mortality, especially in older people and patients with chronic illness. Vaccination against influenza is most effective in preventing the often fatal complications associated with this infection (Murphy, B.R and R.G. Webster 1996). The production of influenza virus on the diploid human cell line MRC-5 has been reported (Herrero-Euribe L et al 1983). However, the titers of influenza virus are prohibitively low.

### Strains of Influenza virus

Present day flu vaccines contain purified Hemagglutinin and neuraminidase of infuenza virus A and B.The 3 viruses that represent epidemiologically important strains are influenza A(H1N1), influenza A(H3N2) and influenza B. The division into A and B types is based on antigenic differences between their nucleoprotein (NP) and matrix (M) protein antigen. The influenza A virus is further subdivided into subtypes based on the antigenic composition (sequence) of hemagglutinin (H1-H15) and neuraminidase (N1-N9) molecules. Representatives of each of these subtypes have been isolated from aquatic birds, which probable are the primordial reservoir of all influenza viruses for avian and mammalian species. Transmission has been shown between pigs and humans and recently (H5N1) between birds and humans.

### Influenza vaccines

Three types of inactivated influenza vaccine are currently used in the world: whole virus, split product and surface antigen or subunit vaccines. These vaccines all contain the surface glycoproteins, haemagglutinin (HA) and neuraminidase (NA), of the influenza virus strains that are expected to circulate in the human population in the upcoming season. These strains, which are incorporated in the vaccine, are grown in embryonated hens' eggs, and the viral particles are subsequently purified before further processing.

The need for the yearly adjustment of influenza vaccines is due to antigen variation caused by processes known as "antigenic drift and shift".

Antigenic drift occurs by the accumulation of a series of point mutations in either the H or N protein of a virus resulting in amino acid substitutions. These substitutions prevent the binding of neutralizing antibodies, induced by previous infection, and the new variant can infect the host. Antigenic shift is the appearance of a new subtype by genetic reassortment between animal and human influenza A viruses. The pandemic strains of 1957 (H2N2) and 1968 (H3N2) are examples of reassorted viruses by which avian H and or N genes were introduced in circulating human viruses, which subsequently could spread among the human population.

Based on the epidemiological surveys by over hundred National Influenza Centers worldwide, the World Health Organization (WHO) yearly recommends the composition of the influenza vaccine, usually in February for the Northern hemisphere, and in September for the Southern hemisphere. This practice limits the time window for production and standardization of the vaccine to a maximum of 9 months.

In case of an urgent demand of many doses of vaccine, for example when a novel subtype of influenza A virus arises by antigenic shift and drift, limited availability of eggs may hamper the rapid production of vaccine. Further disadvantages of this production system are the lack of flexibility, the risk of the presence of toxins and the risks of adventitious viruses, particularly retroviruses, and concerns about sterility. This presents a serious problem in today's practice of influenza vaccine production on embryonated hens' eggs.

Therefor, the use of a cell culture system for influenza vaccine production would be an attractive alternative. Influenza viruses can be grown on a number of primary cells, including monkey kidney, calf kidney, hamster kidney and chicken kidney. Yet, their use for vaccine production is not practical, because of the need to re-establish cultures from these primary cells for each preparation of a vaccine. Therefor, the use of continuous cell lines for influenza vaccine production is an attractive alternative.

The use of culture systems was facilitated by the realization that the proteolytic cleavage of HA in its two subunits (HA1 and HA2), which is required for influenza virus infectivity, can be obtained by the addition of trypsin. Inclusion of trypsin permits replication and plaque formation in Madin-Darby canine kidney (MDCK) cells (Tobita K et al 1975). The MDCK cell line was recently shown to support the growth of influenza virus for vaccine production (Brand R et al 1996, 1997, Palache AM et al 1997). The use of trypsin requires growth of the MDCK cells in serum-free tissue culture medium (MDCK-SF1). However, MDCK cells are currently not approved as a substrate for production of influenza virus.

However, any non-human system for production of influenza vaccines has an inherent drawback, known as 'adaptation'. Human influenza A and B virus both carry mutations in the HA, due to adaptation in embryonated hens' eggs. These mutations result in altered antigenicity (Newman RW, et al 1993, Williams SP and Robertson JS, 1993, Robertson JS et al 1994, Gubareva LV et al 1994, Schild GC et al 1993, Robertson JS et al 1987, Kodihalli S et al 1995). In humans, immunization with vaccines containing an HA bearing an egg-adaption mutation induces less neutralizing antibody to virus that contains a non-egg adapted HA (Newman et al 1993).

Human influenza viruses propagated in canine cells such as MDCK cells also show adaptation, albeit to a lesser extent. Such viruses resemble the original human isolates more closely than egg derived viruses (Robertson JS, 1990. et al).

Furthermore there is evidence that host-specific changes in NA and host-specific phosphorylation patterns of NP can affect the replication of influenza viruses (Schulman JL and Palese P 1977; Sugiara A, Ueda M. 1980; Kistner O et al 1976).

Therefor, it would clearly be advantageous to avoid adaptation or other host-induced changes of influenza virus. It may result in a more homogeneous population of viruses and render the ultimate vaccine more effective.

It is therefor an object of the present invention to provide human cells as a substrate for the production of high titers of influenza virus, suitable for the development of vaccines.

### EXAMPLES

To illustrate the invention, the following examples are provided, not intended to limit the scope of the invention.

### PER.C6 Cell banking

Cell line PER.C6 (deposited under No. 96022940 at the European Collection of Animal Cell Cultures at the Center for Applied Microbiology and Research), or derivatives thereof were used (described in WO 97/00326). Cell lines were banked by a two tier cell bank system. The selected cell line was banked in a research master cell bank (rMCB) which was stored in different locations. From this rMCB working cell banks were prepared as follows: an ampoule of the rMCB was thawed, and the cells were propagated until enough cells are present to freeze the cells by using dry ice. 400-500 ampoules containing 1 ml (1-2x106cells/ml) of rWCB were stored in the vapour phase of a liquid nitrogen freezer.

### PER.C6 preculture

One ampoule containing 5x106 PER.C6 cells of the WCB was thawed in a water bath at 37(C. Cells were rapidly transferred into a 50ml tube and resuspended by adding 9ml of the suspension medium Ex-CellTM 525 (JRH Biosciences, Denver, Pennsylvania) supplemented with 1 x L-Glutamin. After 3 minutes of centrifugation at 1000 rpm, cells were resuspended in a final concentration of 3x105 cells/ml and cultured in a T80cm2 tissue culture flask, at 37(C, 10% C02. Two-three days later, cells were seeded into 490cm2 tissue culture roller bottles (Corning Costar Corporation, Cambridge, USA), with a density of 3x105/ml and cultured in continuous rotation at 1 RPM.

### PER.C6 and MDCK Cell culture

Madin Darby Canine Kidney (MDCK) cells were cultured in Dulbecco's modified Eagle's medium (DMEM, Life Technologies Breda, The Netherlands) containing 10% heat inactivated fetal bovine serum and 1x L-Glutamin (Gibco-BRL), at 37C and 10% C02.

Suspension cultures of PER.C6TM were cultured in Ex-CellTM 525 (JRH Biosciences, Denver, Pennsylvania) supplemented with 1 x L-Glutamin, at 37(C and 10% C02, in stationary cultures in 6 well dishes (Greiner, Alphen aan de Rijn, The Netherlands) or in 490cm2 tissue culture roller bottles (Corning Costar Corporation, Cambridge, USA) during continuous rotation at 1 RPM.

### Immunofluorescence test

Direct immunofluorescence assays for the detection of influenza virus infection were carried out using the IMAGENTM Influenza Virus A and B kit (DAKO, Glostrup, Denmark) according to the standard protocol of the supplier. Samples were viewed microscopically using epifluorescence illumination. Infected cells are characterized by a bright apple-green fluorescence.

### Propidium Iodide staining

Cell pellets were resuspended into 300 µ of cold PBS -0.5% BSA + 5 µ of propidium iodide 50 µg/ml in PBS - FCS - azide solution. Viable and dead cells were then detected via flow cytofluorometric analysis.

### Hemagglutination assay

To 50 µ/l of two fold diluted virus solutions in PBS, 25 l/4l of a 1% suspension of turkey erythrocytes in PBS was added in 96 well microtiter plates and incubated at 4°C for 1h. The hemagglutination pattern was examined, and expressed as hemagglutinating units (HAU). The amount of HAU corresponded to the reciprocal value of the highest virus dilution that showed complete hemagglutination.

PER.C6 cells as permissive cell line for Influenza A virus PER.C6TM is not known for its ability to sustain influenza virus infection and replication. We therefore verified whether PER.C6 cells are permissive for influenza virus infection in comparison with MDCK (Madin Darby Canine Kidney) cells.
The day before infection, 2x105 MDCK cells/well were seeded in 6-well plates. 24 hours later, 4x105 PER.C6/well and MDCK were infected with the H1N1 strain A/Puerto Rico/8/34 (titer 3.6x107 pfu/ml), obtained from Dr. Eric Claas, Dept. of Virology, Leiden University Medical Center, The Netherlands. Infection was performed at various multipliticies of infection (moi's) ranging from of 0.1 to 10 pfu/cell. After about 2 hours of incubation at 37(C, the inoculum was removed and replaced by fresh culture medium. A direct immunofluorescence assay for the detection of influenza virus infection was perfomed 24 and 48 hours post infection. The experiment showed permissivity of PER.C6 for influenza infection, with percentages of positive cells moi-dependent and comparable with MDCK (Table 1).

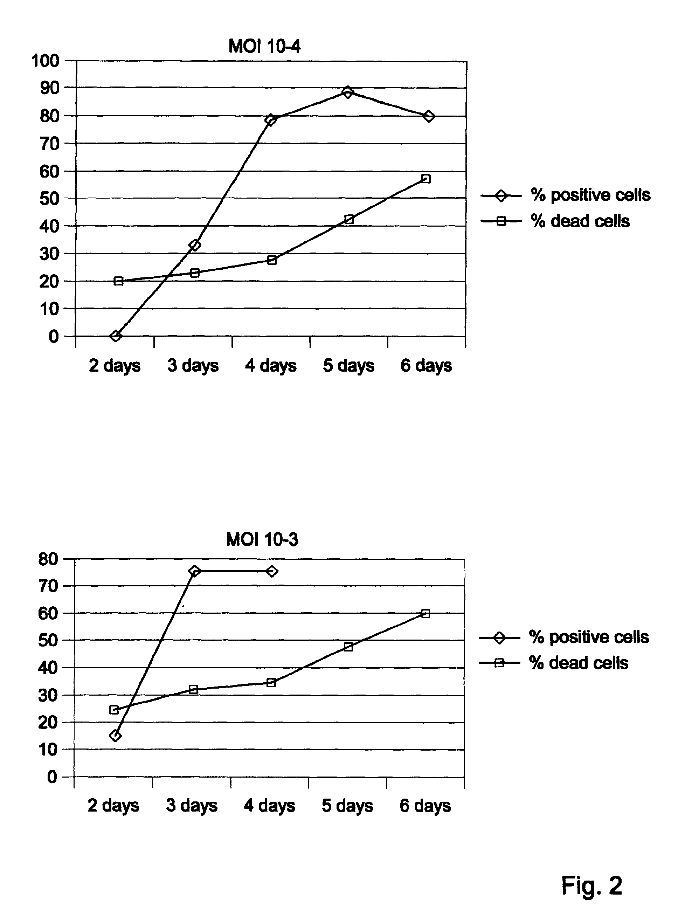

PER.C6 cells as cell line for Influenza A virus propagation We verified whether replication and propagation of influenza virus are supported by PER.C6. The day of infection, PER.C6 cells were seeded in 490cm2 tissue culture roller bottles, with the density of 2x105 cells/ml in a final volume of 40ml, in the presence of 5 µg/ml of trypsine-EDTA (Gibco-BRL). Cells were either, mock inoculated or infected with the H3N2 strain A/Shenzhen/227/95 (titer 1.5x106 pfu/ml), a kind gift from Dr. Eric Claas, Dept. of Virology, Leiden University Medical Center, The Netherlands. Infections were performed at moi 10-4 and 10-3 pfu/cell. After 1 hour of incubation at 37(C, the inoculum was removed by spinning down the cells at 1,500 rpm and resuspending them in fresh culture medium + 5 (g/ml of trypsine-EDTA. Harvest of 1.3 ml of cell suspension was carried out each day, from day 1 to day 6 post-infection. Supernatants were stored at -80(C and used for hemagglutination assays. Cell pellets were used for direct immunofluorescence tests and for propidium iodide staining. (Figure 2).

### Permissivity of PER.C6 for influenza strains

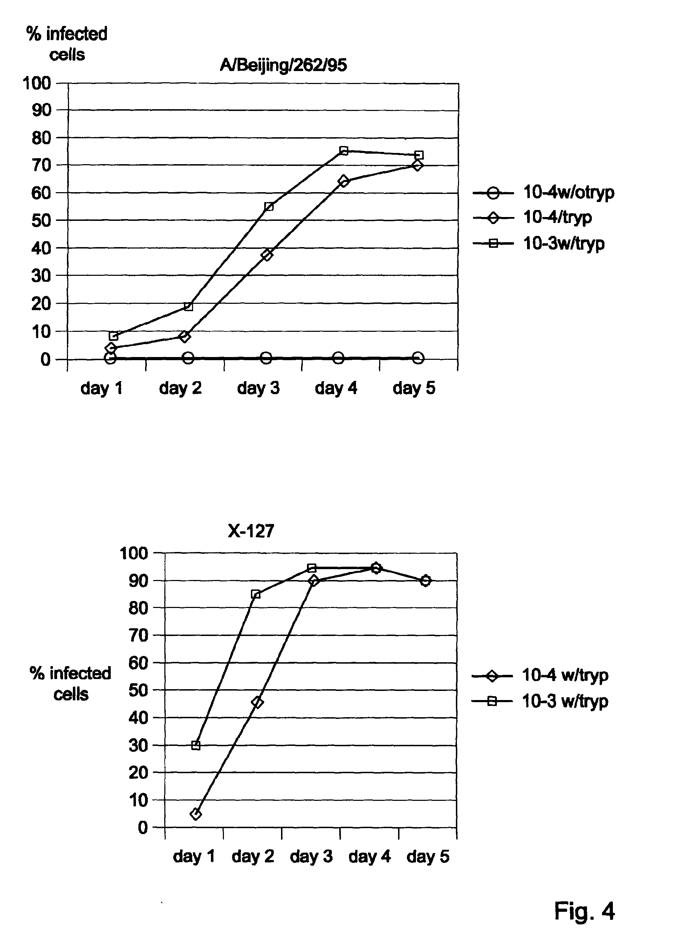

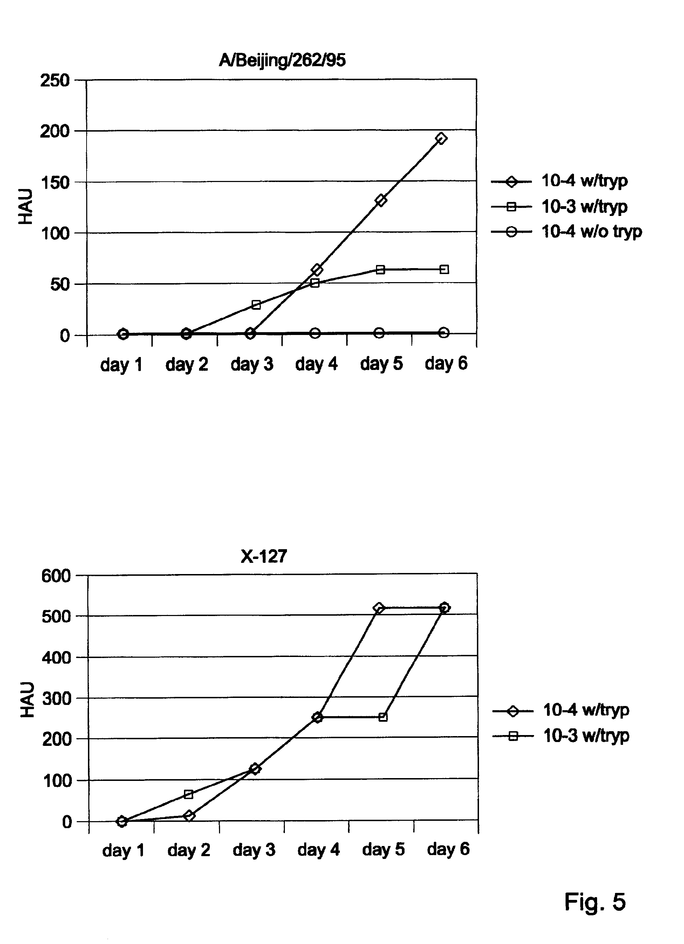

To further investigate the permissivity of PER.C6 for propagation of various influenza strains, we performed an infection by using the H1N1 vaccine strains A/Beijing/262/95 and its reassortant X-127 obtained from the National Institute for Biological Standards and Control (NIBSC), Potters Bar, UK. The day of infection, PER.C6 cells were seeded in 490cm2 tissue culture roller bottles, with the density of approximately 1x10⁶ cells/ml in a final volume of 50ml. Cells were inoculated with 5µl (10-4 dilution) and 50µl (10-3 dilution) of virus in the presence of 5 µg/ml trypsin-EDTA. In order to establish if trypsin was indeed required, one more infection was carried out by inoculating 5µl of the strain A/Beijing/262/95 in the absence of the protease. After approximately 1 hour of incubation at 37°C, the inoculum was removed by spinning down the cells at 1,500 rpm and resuspending them in fresh culture medium ± 5 µg/ml of trypsin-EDTA. At day 2 and day 4 post-infection more trypsin was added to the samples. Harvest of 1.3 ml of cell suspension was carried out from day 1 to day 6 post-infection. Supernatants were stored at -80°C and used for hemagglutination assays and further infections; cell pellets were used for direct immunofluorescence tests. Results obtained with the above mentioned immunofluorescence and hemagglutination assays are shown in fig 4 and fig 5, respectively, illustrating the efficient replication and release of the viruses.

### Infectivity of virus propagated on PER.C6

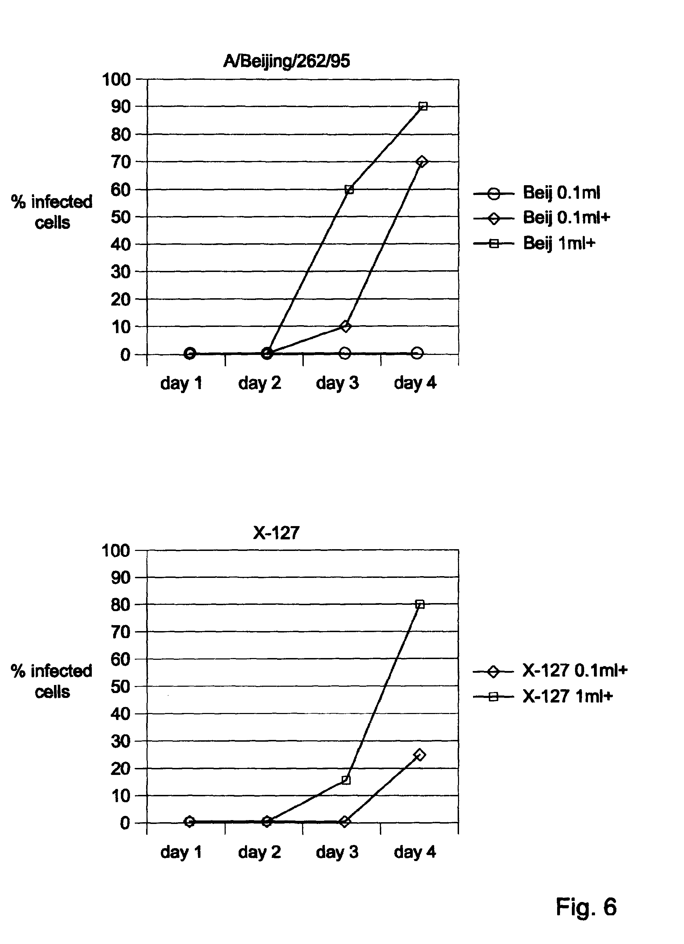

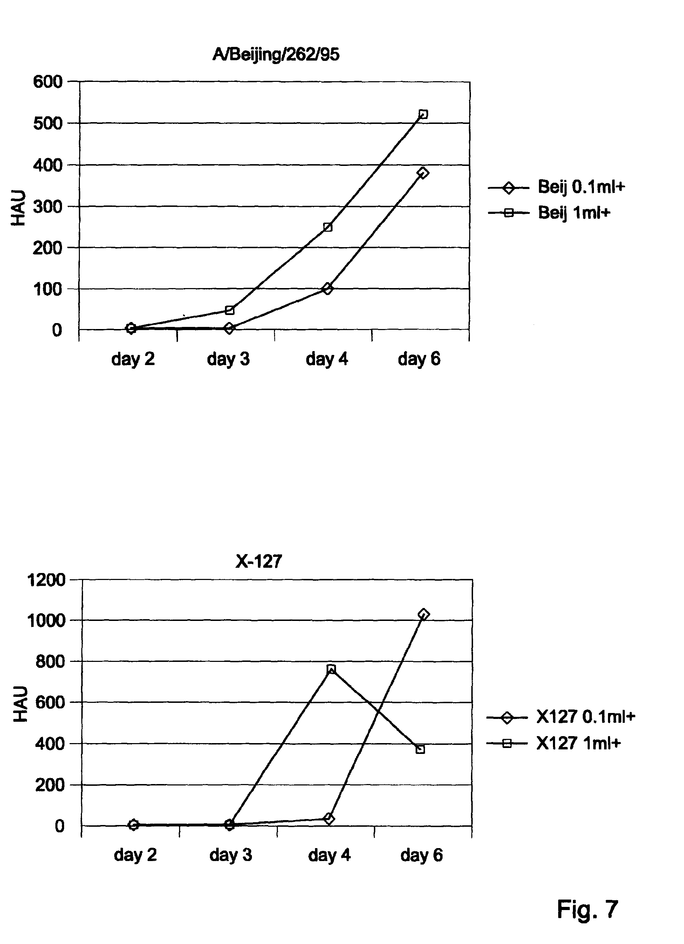

We verified if the viruses grown in PER.C6 were infectious and if adaptation to the cell line could increase virus yields. Virus supernatants derived from PER.C6 infected with the strains A/Beijing/262/95 and its reassortant X-127 (dil.10-3) and harvested at day 6 post-infection, were used. At the day of infection, PER.C6 were seeded in 490cm2 tissue culture roller bottles, with the density of approximately 1x10⁶ cells/ml in a final volume of 50ml. Cells were inoculated with 100 µl and 1 ml of virus supernatant in the presence of 5 µg/ml trypsin-EDTA. In order to establish if trypsin was still required, one more infection was carried out by inoculating 100µl of the strain A/Beijing/262/95 in the absence of the protease. After approximately 1 hour of incubation at 37°C, the inoculum was removed by spinning down the cells at 1,500 rpm and resuspending them in fresh culture medium ± 5 µg/ml of trypsin-EDTA. At day 2 and day 4 post-infection more trypsin was added to the samples. Harvest of 1.3 ml of cell suspension was carried out from day 1 to day 6 post-infection. Supernatants were stored at -80°C and used for hemagglutination assays and further infections; cell pellets were used for direct immunofluorescence tests. Results obtained with the above mentioned immunofluorescence and hemagglutination assays are shown in fig 6 and fig 7. Data obtained with the present experiment showed infectivity of the viruses grown in PER.C6 as well as an increase in virus yields.

### Recovery of virus

Intact virus is recovered from the culture medium by ionexchange chromatography. The virus preparations are further processed to an inactivated surface antigen preparation by formaldehyde inactvation, solubilisation with detergent and ultrafiltration and ultracentrifugation (Bachmayer, H. 1975).

### LEGENDS TO THE FIGURES

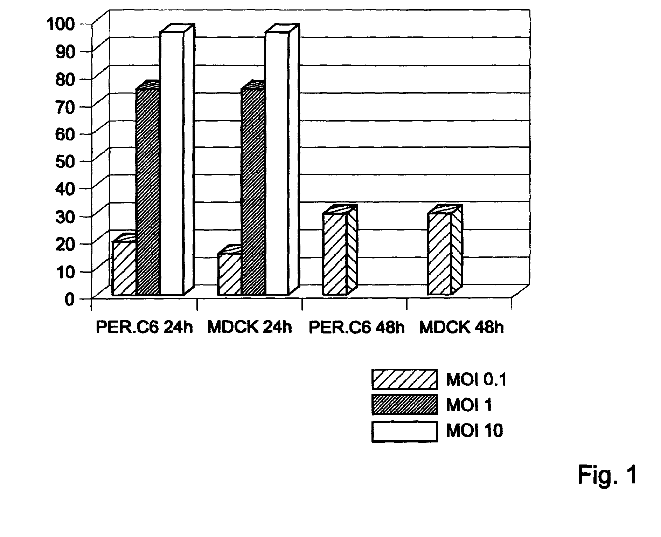

Fig. 1: Percentage of infected cells (positive cells) viewed microscopically after immunofluorescence assay versus percentage of dead cells measured via FACS after propidium iodide staining, at moi's of 10-3 and 10-4. Poor viability of the cells from samples derived from infection at moi 10-3 didn't give rise to reliable data.

Fig. 2: Percentage of infected cells viewed microscopically after immunofluorescence assay. Samples derived from infection at moi 10 and 1, at 48h post infection are not shown, because of full CPE

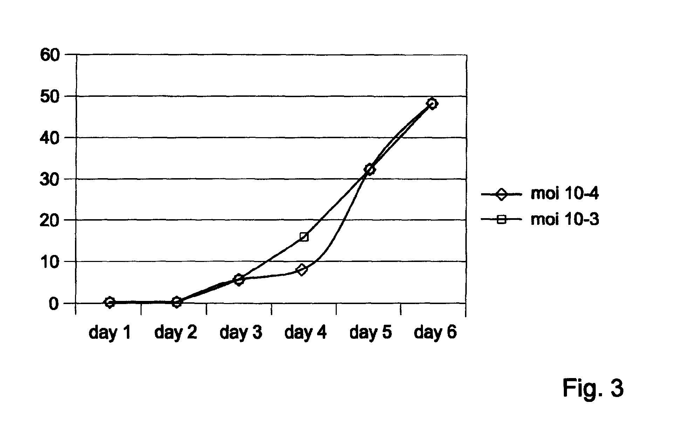

Fig. 3: Kinetics of virus propagation measured in hemagglutinating units (HAU) from day 1 to day 6 after infection.

Fig.4: Percentage of infected cells (positive cells) viewed microscopically after immunofluorescence assay.

Fig. 5: Kinetics of virus propagation measured in hemagglutinating units (HAU) from day 1 to 6 after infection.

Fig. 6: Percentage of infected cells (positive cells) viewed microscopically after immunofluorescence assay.

Fig. 7: Kinetics of virus propagation measured in hemagglutinating units (HAU) from day 2 to 6 after infection.

### REFERENCES

Bachmayer H. Selective solubilisation of hemagglutinin and neuraminidase from influenza virus. Intervirology 1975; 5:260-272.

Brands R, Palache AM, van Scharrenburg GJM. Madin Darby Canine Kidney (MDCK)-cells for the produvtion of inactivated influenza subunit vaccin. Safety characteristics and clinical results in the elderly. In: Brown LE, Hampson EW, Webster RG, editors. Option for the control of influenza III. Amsterdam Elsevier, 1996. P. 683-693.

Brands R, Palache AM, van Scharrenburg GJM. Development of influenza subunit vaccine produced using mammalian cell culture technology. In. Carrondo MJT, Griffths B, Moreira JLP, editors. Animal cell technology: from vaccines to genetic medicine. Dordrecht: Kluwer Academic Publishers, 1997:165-167.

Gubareva LV, Wood JM, Meyer WJ, Katz JM, Robertson JS, Major D, Webster RG. Codominant mixtures of viruses in strains of influenza virus due to host cell variation. Virol. 1994; 199: 89-97.

Herrero-Euribe L et al. Replication of Influenza A and B viruses in human diploid cells. J. Gen. Virol. 1983; 64: 471-475.

Kodihalli S, Justewicz DM, Gubareva LV, Webster RG. Selection of a single amino acid substitution in the hemagglutinin molecule by chicken eggs can render influenza A virus (H3) candidate vaccine ineffective. J. Virol. 1995;69:4888-4897.

Kirstner O, Muller K, Scholtissek C. Differential phosphorylatian of the nucleoprotein of influenza A viruses. J. Gen. Virol. 19989;70:2421-2431.

Murphy BR and Webster RG. Orthomyxoviruses. In: Fields Virology, chapter 46, 1397. Eds.B.N. Fields, D.M. Knipe, P.M. Howley, et al. Lippincott-Raven Publishers, Philadelphia 1996.

Newman RW, Jenning R Major DL, Robertson JS, Jenkins R, Potter CW, Burnett I, Jewes L, Anders M, Jackson D, Oxford JS. Immune response of human volunteers an animals to vaccination with egg grown influenza A (H1N1) virus is influenced by three amino acid substitutions in the hemagglutinin molecule. Vaccine 1993;11:400-406.

Palache AM, Brands R, van Scharrenburg GJM. Immunogenecity and reactogenecity of influenza subunit vaccines produced in MDCK cells or fertilized chicken eggs. J. Infet. Dis. 1977;176:S20-S23.

Robertson JS, Cook P, Nicolson C, Newman R,Wood JM. Mixed populations in influenza vaccine strains. Vaccine 1994; 12:1317-1320.

Robertson JS,Bootman JS,Nicolson C, Mjor D, Robertson EW, Wood JM. The hemagglutinin of influenza B virus present in clinical material is a single species identical to that of mammalian cell grown-virus. Virol. 1990; 179:35-40

Robertson JS, Bootman JS, newman R, Oxford JS, Daniels RS, Webster RG, Schild GC. Structural changes in the hemagglutinin wich accompany egg adaptation of an influenza A(H1N1) virus. Virol. 1987;160:31-37.

Schild GC, Oxford JS, de Jong JC, Webster RG. Evidence for host-cell selection of influenza virus antigenic variants. Nature 1983; 303:706-709.

Schulman JL, Palese P. Virulence factors of influenza A viruses: WSN virus neuraminidase required for plaque production in MDBK cells. J. Virol. 1977;24:170-176.

Sugiara A, Ueda M. Neurovirulence of influenza virus in mice. I. Neurovirulence of recombinants between virulent and avirulent virus strains. Virol 1980;101:440-449.,495,271).

Tobita K, Sugiura A, Enomoto C, Furuyama M. Plaque assay and primary isolation of influenza A viruses in an established line of canine Kidney cells (MDCK) in the presence of trypsin. Med.Microbiol. Immunol. 1975;162:9-14.

Williams SP, Robertson JS. Analysis of restriction to the growth of non-egg-adapted human influenza in eggs. Virol. 1993;196:660665.

## Claims

1. A method for producing a virus and/or viral proteins other than adenovirus or adenoviral proteins for use as a vaccine comprising providing a cell with at least a sequence encoding at least one gene product of the E1 gene or a functional derivative thereof of an adenovirus, providing said cell with a nucleic acid encoding said virus and/or said viral proteins, culturing said cell in a suitable medium and allowing for expression of said virus and/or said viral proteins and harvesting said virus and/or viral proteins from said medium and/or said cell.

2. A method according to claim 1, wherein said cell is a human primary cell.

3. A method according to claim 1 or 2, wherein said primary cell is immortalised by a gene product of said E1 gene.

4. A method according to any one of claims 1-3, wherein said cell is derived from a human embryonic retinoblast.

5. A method according to any one of claims 1-4, wherein said sequence encoding at least one gene product of the E1 gene is present in the genome of said human cell.

6. A method according to any one of claims 1-5, wherein said cell does not produce adenoviral structural proteins.

7. A method according to any one of the aforegoing claims, wherein said cell further comprises a sequence encoding E2A or a functional derivative or analogue or fragment thereof.

8. A method according to any one of the aforegoing claims wherein said sequence encoding E2A or a functional derivative or analogue or fragment thereof is present in the genome of said human cell.

9. A method according to any one of claims 7 or 8, wherein said E2A encoding sequence encodes a temperature sensitive mutant E2A.

10. A method according to any one of the aforegoing claims, whereby said human cell comprises no other adenoviral sequences.

11. A method according to any one of the aforegoing claims, wherein said human cell is capable of growing in suspension.

12. A method according to anyone of the aforegoing claims wherein said human cell can be cultured in the absence of serum.

13. A method according to any one of the aforegoing claims wherein said human cell is PER.C6 as deposited under ECACC no. 96022940 or a derivative thereof.

14. A method according to any one of claims 1-13, wherein said virus and/or said viral proteins comprise a protein that undergoes post-translational and/or peritranslational modifications.

15. A method according to claim 14 wherein said modifications comprise glycosylation.

16. A method according to any one of the aforegoing claims wherein said viral proteins comprise at least one of an Influenza virus neuramidase and/or a haemagglutinin.

17. A method according to any one of claims 1-16, wherein said virus is an enterovirus, such as rhinovirus, aphtovirus, or poliomyelitisvirus.

18. A method according to any one of claims 1-16, wherein said virus is a herpesvirus, such as herpes symplex virus, pseudorabies virus or bovine herpes virus.

19. A method according to any one of claims 1-16, wherein said virus is an orthomyxovirus, such as influenza virus, a paramyxovirus, such as newcastle disease virus, respiratory syncitio virus, mumps virus or a measles virus.

20. A method according to any one of claims 1-16, wherein said virus is a retrovirus, such as human immunedeficiency virus or wherein said virus is a parvovirus or a papovavirus.

21. A method according to any one of claims 1-16, wherein said virus is a rotavirus or a coronavirus, such as transmissable gastroenteritisvirus or a flavivirus, ,such as tick-borne encephalitis virus or yellow fever virus.

22. A method according to any one of claims 1-16, wherein said virus is a togavirus, such as rubella virus or eastern-, western-, or venezuelean equine encephalomyelitis virus.

23. A method according to any one of claims 1-16, wherein said virus is a hepatitis causing virus, such as hepatitis A or hepatitis B virus.

24. A method according to any one of claims 1-16, wherein said virus is a pestivirus, such as hog cholera virus or a rhabdovirus, such as rabies virus.

25. Use of a human cell having a sequence encoding at least one E1 protein of an adenovirus or a functional derivative, homologue or fragment thereof in its genome which cell does not produce structural adenoviral proteins for the production of a virus or at least one viral protein for use in a vaccine.

26. Use according to claim 25, wherein said human cell is derived from a primary cell.

27. Use according to claim 25 or 26, wherein said human cell is a PER.C6 cell or a derivative thereof.

28. Use according to claim 25-27, wherein said cell further comprises a sequence encoding E2A or a functional derivative or analogue or fragment thereof in its genome.

29. Use according to claim 28, wherein said E2A is temeperature sensitive.

30. A virus or a viral protein for use in a vaccine obtainable by a method according to any one of claims 1-24 or by a use according to any one of claims 25-29, said virus or said viral being free of any non-human mammalian protenaceous material.

31. A human cell having a sequence encoding at least one E1 protein of an adenovirus or a functional derivative, homologue or fragment thereof in its genome, which cell does not produce structural adenoviral proteins and having a nucleic acid encoding a virus or at least one non-adenoviral viral protein.

32. A human cell according to claim 31 which is derived from PER.C6 as deposited under ECACC no. 96022940.

33. A human cell according to claim 31-32, which further comprises a sequence encoding E2A or a functional derivative or analogue or fragment thereof in its genome.

34. A human cell according to claim 33, wherein said E2A is temperature sensitive.
